# EUROPEAN PATENT SPECIFICATION

(11) **EP 4 590 258 B1**
(45) Date of publication and mention of the grant of the patent: **23.09.2026**
(21) Application number: 23802103.4
(22) Date of filing: 12.10.2023
(51) Int. Cl.: A61K 8/31, A61K 8/73, A61Q 1/02, C08B 31/08

(54) **SKIN CARE FORMULATION**
HAUTPFLEGEFORMULIERUNG
FORMULATION DE SOIN DE LA PEAU

(30) Priority: 18.10.2022 US 202263416952 P
(43) Date of publication of application: 30.07.2025
(73) Proprietor: Dow Silicones Corporation, Midland, MI 48686-0994 (US); Rohm and Haas Company, Collegeville, PA 19426 (US)
(72) Inventor: CORTES-CLERGET, Margery, Auburn, Michigan 48611 (US); COURTEMANCHE, Marc-Andre, Auburn, Michigan 48611 (US); XU, Wenjun, Collegeville, Pennsylvania 19426 (US); DIHANG, Helene, 7180 Seneffe (BE); VOGEL, Jason, Freeland, Michigan 48623 (US)
(74) Representative: Beck Greener LLP
(86) International application number: PCT/US2023/076635
(87) International publication number: WO 2024/086470

(56) References cited:
- DATABASE GNPD [online] MINTEL; 26 July 2022 (2022-07-26), ANONYMOUS: "Slim Mascara", XP093118959, retrieved from https://www.gnpd.com/sinatra/recordpage/9768612/ Database accession no. 9768612
- DATABASE GNPD [online] MINTEL; 16 September 2022 (2022-09-16), ANONYMOUS: "Skincare Foundation SPF 27", XP093118958, retrieved from https://www.gnpd.com/sinatra/recordpage/9914990/ Database accession no. 9914990
- CHESTER THOMAS L. ET AL: "Determination of Maltodextrin in Psyllium-Based Bulk Laxatives by in Situ Silylation and Supercritical Fluid Chromatography", ANALYTICAL CHEMISTRY, vol. 67, no. 7, 1 April 1995 (1995-04-01), US, pages 1290 - 1292, XP093118839, ISSN: 0003-2700, DOI: 10.1021/ac00103a022

## Description

The present invention relates to a skin care formulation. In particular, the present invention relates to a skin care formulation including a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 1 to 24; wherein the functionalized maltodextrin has a degree of substitution of -Si(R¹)₃ groups, DS, of 1.7 to 3; and wherein the functionalized maltodextrin is free of vinylic carbon.

Many skin care compositions, including pigmented cosmetics (e.g., foundations, concealers, lipsticks, mascaras) and sunscreens, have been developed for longer wear and transfer resistance properties. These properties are often accomplished by the use of compositions that form a film after application. Such compositions generally contain volatile solvents, which evaporate on contact with the skin or other keratinous tissue, leaving behind a layer comprising waxes and/or resins, pigments, fillers, and actives. Conventional film formers having desirable esthetic characteristics, for example, polyvinyl pyrrolidone, acrylates, acrylamides and copolymers thereof all tend not to offer the sustainable solution brand owners and consumers now seek.

Mintel GNPD record ID 9768612 "Slim Mascara", Jul 2022 shows a low smudging (ie. transfer resistant) mascara comprising isododecane as carrier and maltodextrin. Maltodextrin is known in the art as a film-forming ingredient. Mintel GNPD record ID 9914990 "Skincare Foundation SPF 27", Sep 2022 shows a transfer resistant foundation comprising isododecane as carrier and maltodextrin.

Accordingly, there remains a need for new film forming ingredients that facilitate the need for improved long-wearing skin care formulations while simultaneously having an increased bio carbon content when compared to conventional film forming ingredients.

The present invention provides a skin care formulation, comprising: a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 1 to 24; wherein the functionalized maltodextrin has a degree of substitution of -Si(R¹)₃ groups, DS, of 1.7 to 3; and wherein the functionalized maltodextrin is free of vinylic carbon.

### DETAILED DESCRIPTION

We have surprisingly found a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 1 to 24; wherein the functionalized maltodextrin has a degree of substitution of -Si(R¹)₃ groups, DS, of 1.7 to 3; and wherein the functionalized maltodextrin is free of vinylic carbon. The film forming polymer of the present invention is a biobased material. Moreover, we have surprisingly found that the film forming polymer of the present invention provides water and sebum resistance facilitating long wear products such as color cosmetics, sun care formulations and antiperspirant/deodorants with desirable aesthetic characteristics.

Unless otherwise indicated, ratios, percentages, parts, and the like are by weight.

The term "dextrose equivalent, DE" as used herein and in the appended claims refers to the degree of starch hydrolysis, specifically, the reducing value of a starch hydrolysate material compared to the reducing value of an equal weight of dextrose, expressed as percent, dry basis, as measured by the Lane and Eynon method described in Standard Analytical Method E-26, Corn Refiners Association, 6th edition, 1977, E-26, pp. 1-3. For example, a maltodextrin with a DE of 10 would have 10% of the reducing power of dextrose which has a DE of 100.

The term "vinylic carbon" as used herein and in the appended claims refers to a carbon that is involved in a double bond with another carbon.

The term "free of vinylic carbon" as used herein and in the appended claims in reference to the functionalized maltodextrin means that the functionalized maltodextrin contains less than the detectable limit of vinylic carbon.

The term "dermatologically acceptable" as used herein and in the appended refers to ingredients typically used in personal care compositions and is intended to underscore that materials that are toxic when present in the amounts typically found in personal care compositions are not contemplated as part of the present invention.

The term "aesthetic characteristics" as used herein and in the appended claims in reference to a skin care formulation refers to visual and tactile sensory properties (e.g., smoothness, tack, lubricity, texture, color, clarity, turbidity, uniformity).

Preferably, the skin care formulation of the present invention is selected from the group consisting of a color cosmetic formulation, a sun care formulation, a hand cream, a skin cream, a face cream, an anti-perspirant formulation and a deodorant formulation. More preferably, the skin care formulation of the present invention is selected from the group consisting of a color cosmetic formulation, a sun care formulation, an anti-perspirant and a deodorant formulation. Most preferably, the skin care formulation of the present invention is a color cosmetic formulation.

Preferably, the skin care formulation of the present invention is provided in a product form selected from the group consisting of a cream, a non-aqueous solution, an emulsion, an oil, an ointment, a paste, a gel, a lotion, a milk, a foam, a stick and a suspension. More preferably, the skin care formulation of the present invention is provided as an emulsion.

Preferably, the skin care formulation of the present invention, comprises: a film forming polymer (preferably, 0.1 to 100 wt% (more preferably, 0.5 to 50 wt%; still more preferably, 1 to 25 wt%; most preferably, 1.5 to 7.5 wt%), based on weight of the skin care formulation, of the film forming polymer), wherein the film forming polymer is a functionalized maltodextrin comprising a maltodextrin base polymer functionalized with - Si(R¹)₃ groups; wherein each R¹ is independently a C₁₋₁₀ linear or branched, saturated alkyl group; wherein the maltodextrin base polymer has a dextrose equivalent, DE, of 1 to 24 (preferably, 1 to 20; more preferably, 1 to 18; still more preferably, 1 to 15; most preferably, 1); wherein the functionalized maltodextrin has a degree of substitution of -Si(R¹)₃ groups, DS, of 1.7 to 3 (preferably, 1.8 to 3; more preferably, 2 to 3; most preferably, 2.1 to 2.8); and wherein the functionalized maltodextrin is free of vinylic carbon; optionally, a dermatologically acceptable carrier (preferably, 0 to 98 wt% (more preferably, 30 to 92 wt%; still more preferably, 35 to 90 wt%; most preferably, 40 to 80 wt%), based on weight of the skin care formulation, of the dermatologically acceptable carrier); optionally, a color ingredient (preferably, 0 to 90 wt% (more preferably, 0.01 to 65 wt%; still more preferably, 1 to 50 wt%; most preferably, 5 to 25 wt%), based on weight of the skin care formulation, of the color ingredient); and optionally, a sun care active (preferably, 0 to 70 wt% (more preferably, 0.1 to 65 wt%; still more preferably, 5 to 60 wt%; most preferably, 10 to 25 wt%), based on weight of the skin care formulation, of the sun care active). More preferably, the skin care formulation of the present invention, comprises: a film forming polymer (preferably, 0.1 to 100 wt% (more preferably, 0.5 to 50 wt%; still more preferably, 1 to 25 wt%; most preferably, 1.5 to 7.5 wt%), based on weight of the skin care formulation, of the film forming polymer), wherein the film forming polymer is a functionalized maltodextrin comprising a maltodextrin base polymer functionalized with -Si(R¹)₃ groups; wherein the -Si(R¹)₃ groups are linked to the maltodextrin base polymer through a C-O-Si bond; wherein each R¹ is independently a C₁₋₁₀ linear or branched, saturated alkyl group; wherein the maltodextrin base polymer has a dextrose equivalent, DE, of 1 to 24 (preferably, 1 to 20; more preferably, 1 to 18; still more preferably, 1 to 15; most preferably, 1); wherein the functionalized maltodextrin has a degree of substitution of -Si(R¹)₃ groups, DS, of 1.7 to 3 (preferably, 1.8 to 3; more preferably, 2 to 3; most preferably, 2.1 to 2.8); and wherein the functionalized maltodextrin is free of vinylic carbon; optionally, a dermatologically acceptable carrier (preferably, 0 to 98 wt% (more preferably, 30 to 92 wt%; still more preferably, 35 to 90 wt%; most preferably, 40 to 80 wt%), based on weight of the skin care formulation, of the dermatologically acceptable carrier); optionally, a color ingredient (preferably, 0 to 90 wt% (more preferably, 0.01 to 65 wt%; still more preferably, 1 to 50 wt%; most preferably, 5 to 25 wt%), based on weight of the skin care formulation, of the color ingredient); and optionally, a sun care active (preferably, 0 to 70 wt% (more preferably, 0.1 to 65 wt%; still more preferably, 5 to 60 wt%; most preferably, 10 to 25 wt%), based on weight of the skin care formulation, of the sun care active).

Preferably, the skin care formulation of the present invention, comprises 0.1 to 100 wt% (preferably, 0.5 to 50 wt%; more preferably, 1 to 25 wt%; most preferably, 1.5 to 7.5 wt%), based on weight of the skin care formulation, of a film forming polymer; wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group (preferably, a methyl group, an ethyl group, a propyl group, a butyl group and a pentyl group; more preferably, a methyl group, an ethyl group, a propyl group and a butyl group; still more preferably, a methyl group, an ethyl group and a propyl group; yet more preferably, a methyl group and an ethyl group; most preferably, a methyl group); wherein the maltodextrin base polymer has a dextrose equivalent, DE, of 1 to 24 (preferably, 1 to 20; more preferably, 1 to 18; still more preferably, 1 to 15; most preferably, 1); wherein the functionalized maltodextrin has a degree of substitution of - Si(R¹)₃ groups, DS, of 1.7 to 3 (preferably, 1.8 to 3; more preferably, 2 to 3; most preferably, 2.1 to 2.8); and wherein the functionalized maltodextrin is free of vinylic carbon. More preferably, the skin care formulation of the present invention, comprises 0.1 to 100 wt% (preferably, 0.5 to 50 wt%; more preferably, 1 to 25 wt%; most preferably, 1.5 to 7.5 wt%), based on weight of the skin care formulation, of a film forming polymer; wherein the film forming polymer is a functionalized maltodextrin comprising a maltodextrin base polymer functionalized with -Si(R¹)₃ groups; wherein the -Si(R¹)₃ groups are linked to the maltodextrin base polymer through a C-O-Si bond; wherein each R¹ is independently a C₁₋₁₀ linear or branched, saturated alkyl group (preferably, a methyl group, an ethyl group, a propyl group, a butyl group and a pentyl group; more preferably, a methyl group, an ethyl group, a propyl group and a butyl group; still more preferably, a methyl group, an ethyl group and a propyl group; yet more preferably, a methyl group and an ethyl group; most preferably, a methyl group); wherein the maltodextrin base polymer has a dextrose equivalent, DE, of 1 to 24 (preferably, 1 to 20; more preferably, 1 to 18; still more preferably, 1 to 15; most preferably, 1); wherein the functionalized maltodextrin has a degree of substitution of -Si(R¹)₃ groups, DS, of 1.7 to 3 (preferably, 1.8 to 3; more preferably, 2 to 3; most preferably, 2.1 to 2.8); and wherein the functionalized maltodextrin is free of vinylic carbon.

Preferably, the maltodextrin base polymer has a dextrose equivalent, DE, of 1 to 24 (preferably, 1 to 20; more preferably, 1 to 18; still more preferably, 1 to 15; most preferably, 1). More preferably, the maltodextrin base polymer has a dextrose equivalent, DE, of 1 to 24 (preferably, 1 to 20; more preferably, 1 to 18; still more preferably, 1 to 15; most preferably, 1); wherein the maltodextrin base polymer is a straight or branched chain maltodextrin polymer comprising a plurality of glucose structural units. Most preferably, the maltodextrin base polymer has a dextrose equivalent, DE, of 1 to 24 (preferably, 1 to 20; more preferably, 1 to 18; still more preferably, 1 to 15; most preferably, 1); wherein the maltodextrin base polymer is a straight or branched chain maltodextrin polymer comprising a plurality of glucose structural units; wherein 90 to 100 mol% (preferably, 92 to 100 mol%; more preferably, 93 to 100 mol%; most preferably, 94.5 to 100 mol%) of the glucose structural units are connected by α-1,4 linkages and 0 to 10 mol% (preferably, 0 to 8 mol%; more preferably, 0 to 7 mol%; most preferably, 0 to 5.5 mol%) of the glucose structural units are connected by α-1,6 linkages.

Preferably, the maltodextrin base polymer contains less than 0.01 wt%, based on weight of the maltodextrin base polymer, of alternan. More preferably, the maltodextrin base polymer contains less than 0.001 wt%, based on weight of the maltodextrin base polymer, of alternan. Most preferably, the maltodextrin base polymer contains less than the detectable limit of alternan.

Preferably, < 0.1 mol% (preferably, < 0.01 mol%; more preferably, < 0.001 mol%; most preferably, < detectable limit) of the glucose structural units in the maltodextrin base polymer are connected by β-1,4 linkages.

Preferably, < 0.1 mol% (preferably, < 0.01 mol%; more preferably, < 0.001 mol%; most preferably, < detectable limit) of the glucose structural units in the maltodextrin base polymer are connected by β-1,3 linkages.

Preferably, the skin care formulation of the present invention, comprises 0 to 98 wt% (preferably, 30 to 92 wt%; more preferably, 35 to 90 wt%; most preferably, 40 to 80), based on weight of the skin care formulation, of a dermatologically acceptable carrier. More preferably, the skin care formulation of the present invention, comprises 0 to 98 wt% (preferably, 30 to 92 wt%; more preferably, 35 to 90 wt%; most preferably, 40 to 80), of a dermatologically acceptable carrier; wherein the dermatologically acceptable carrier is selected from the group consisting of water; glycols (e.g., ethylene glycol, propylene glycol, butylene glycol, pentylene glycol, hexylene glycol, dipropylene glycol, ethoxydiglycol); C₁₋₁₀ straight or branched chain alcohols (e.g., methyl alcohol, ethyl alcohol, propyl alcohol, isopropyl alcohol, butyl alcohol, 2-butoxyethanol); ketones (e.g., acetone); acetates (e.g., methyl acetate); butyl cellusolve; dimethicones; polydimethylsiloxanes; alkanes (e.g., isododecane, isohexane); alkanoates (e.g., methyl undecanoate), dermatologically acceptable hydrophobic ester oils (e.g., caprylic capric triglycerides); dicaprylyl carbonate; alkyl benzoates (e.g., C₁₂₋₁₅ alkyl benzoate); hemisqualane; dioctylether; keto acids (e.g., levulinic acid) and mixtures thereof. Still more preferably, the skin care formulation of the present invention, comprises 0 to 98 wt% (preferably, 30 to 92 wt%; more preferably, 35 to 90 wt%; most preferably, 40 to 80), of a dermatologically acceptable carrier; wherein the dermatologically acceptable carrier is selected to be capable of evaporating upon application of the skin care formulation to the skin (preferably, human skin). Most preferably, the skin care formulation of the present invention, comprises 0 to 98 wt% (preferably, 30 to 92 wt%; more preferably, 35 to 90 wt%; most preferably, 40 to 80), of a dermatologically acceptable carrier; wherein the dermatologically acceptable carrier includes isododecane; and wherein the dermatologically acceptable organic carrier is selected to be capable of evaporating upon application of the skin care formulation to the skin (preferably, human skin).

Preferably, the skin care formulation of the present invention comprises 0 to 90 wt% (preferably, 0.01 to 65 wt%; more preferably, 1 to 50 wt%; preferably, 5 to 25 wt%), based on weight of the skin care formulation, of a color ingredient. More preferably, the skin care formulation of the present invention comprises 0 to 90 wt% (preferably, 0.01 to 65 wt%; more preferably, 1 to 50 wt%; preferably, 5 to 25 wt%), based on weight of the skin care formulation, of a color ingredient; wherein the color ingredient is selected from the group consisting of inorganic pigments, organic pigments, aqueous pigment dispersions and mixtures thereof. Still more preferably, the skin care formulation of the present invention comprises 0 to 90 wt% (preferably, 0.01 to 65 wt%; more preferably, 1 to 50 wt%; preferably, 5 to 25 wt%), based on weight of the skin care formulation, of a color ingredient; wherein the color ingredient is selected from the group consisting of Ext. D&C Yellow No. 2, Ext. D & C Violet No. 2, FD&C Red No. 4, FD&C Red No. 40, FD&C Yellow No. 5, FD&C Yellow No. 6, FD&C Green No. 3, FD&C Blue No. 1, D&C Yellow No. 7, D&C Yellow No. 8, D&C Yellow No. 10, D&C Yellow No. 11, D&C Violet No. 2, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 34, D&C Red No. 33, D&C Red No. 36, D&C Green No. 5, D&C Green No. 6, D&C Green No. 8, D&C Blue No. 4, D&C Orange No. 4, D&C Orange No. 5, D&C Orange No. 10, D&C Orange No. 11, D&C Brown No. 1, Aluminum powder, Annatto, Bismuth citrate, Bismuth Oxychloride, Bronze powder, Caramel, Carmine, β-Carotene, Chromium hydroxide green, Chromium oxide green, Copper chlorophyllin, Copper powder, Dihydroxyacetone, Ferric Ammonium ferrocyanide, Ferric ferrocyanide, Guanine, Iron oxide, Manganese Violet, Mica, Silver, Titanium Dioxide, Ultramarine, Zinc Oxide and mixtures thereof. Still more preferably, the skin care formulation of the present invention comprises 0 to 90 wt% (preferably, 0.01 to 65 wt%; more preferably, 1 to 50 wt%; preferably, 5 to 25 wt%), based on weight of the skin care formulation, of a color ingredient; wherein the color ingredient includes at least one iron oxide. Most preferably, preferably, the skin care formulation of the present invention comprises 0 to 90 wt% (preferably, 0.01 to 65 wt%; more preferably, 1 to 50 wt%; preferably, 5 to 25 wt%), based on weight of the skin care formulation, of a color ingredient; wherein the color ingredient includes a mixture of iron oxides.

Preferably, the color cosmetic formulation of the present invention, comprises 0 to 70 wt% (more preferably, 0.1 to 65 wt%; still more preferably, 5 to 60 wt%; most preferably, 10 to 25 wt%), based on weight of the skin care formulation, of a sun care active. More preferably, the skin care formulation of the present invention comprises 0 to 70 wt% (more preferably, 0.1 to 65 wt%; still more preferably, 5 to 60 wt%; most preferably, 10 to 25 wt%), based on weight of the skin care formulation, of a sun care active; wherein the suncare active is a UV radiation absorbing agent. Still more preferably, the skin care formulation of the present invention, comprises 0 to 70 wt% (more preferably, 0.1 to 65 wt%; still more preferably, 5 to 60 wt%; most preferably, 10 to 25 wt%), based on weight of the skin care formulation, of a sun care active; wherein the suncare active is a UV radiation absorbing agent selected from the group consisting of physical blockers (e.g., red petrolatum, titanium dioxide, zinc oxide); chemical absorbers (e.g., 1-(4-methoxyphenol)-3-(4-tert-butylphenyl)propane-1,3-dione (INCI: Butyl Methoxydibenzoylmethane); 2-hydroxy-4-methoxybenzophenone (INCI: Benzophenone-3); dioxybenzone; sulisobenzone; menthyl anthranilate; para-aminobenzoic acid; amyl paradimethylaminobenzoic acid; octyl para-dimethylaminobenzoate; ethyl 4-bis (hydroxypropyl) para-aminobenzoate; polyethylene glycol (PEG-25) para-aminobenzoate; ethyl 4-bis (hydroxypropyl) aminobenzoate; diethanolamine para-methyoxycinnamate; 2-ethoxyethyl para-methoxycinnamate; ethylhexyl para-methoxycinnamate; octyl para-methoxycinnamate; isoamyl para-methoxycinnamate; 2-ethylhexyl-2-cyano-3,3-diphenyl-acrylate; 2-ethylhexyl-2-cyano-3,3-diphenyl-2-propenoate (INCI: octocrylene); 2-ethylhexyl-2-hydroxybenzoate (INCI: Ethylhexyl Salicylate); homomenthyl salicylate; glyceryl aminobenzoate; triethanolamine salicylate; digalloyl trioleate; lawsone with dihydroxyacetone; 2-phenylbenzimidazole-5-sulfonic acid; 4-methylbenzylidine camphor; avobenzone; triazines; benzotriazoles; vinyl group-containing amides; cinnamic acid amides; sulfonated benzimidazoles); 3,3,5-trimethylcyclohexyl 2-hydroxybenzoate (INCI: Homosalate)); and mixtures thereof. Yet more preferably, the skin care formulation of the present invention, comprises 0 to 70 wt% (more preferably, 0.1 to 65 wt%; still more preferably, 5 to 60 wt%; most preferably, 10 to 25 wt%), based on weight of the skin care formulation, of a sun care active; wherein the suncare active is a UV radiation absorbing agent comprises a mixture of UV radiation absorbing agents. Most preferably, the skin care formulation of the present invention, comprises 0 to 70 wt% (more preferably, 0.1 to 65 wt%; still more preferably, 5 to 60 wt%; most preferably, 10 to 25 wt%), based on weight of the skin care formulation, of a sun care active; wherein the suncare active is a UV radiation absorbing agent is a mixture of UV absorbing agents including at least one of titanium dioxide; 1-(4-methoxyphenol)-3-(4-tert-butylphenyl)propane-1,3-dione; 2-ethylhexyl-2-hydroxybenzoate; 2-ethyhexyl-2-cyano-3,3-diphenyl-2-propenoate; 2-hydroxy-4-methoxybenzophenone and 3,3,5-trimethylcyclohexyl 2-hydroxybenzoate.

Preferably, the skin care formulation of the present invention, optionally, further comprises an additive. More preferably, the skin care formulation of the present invention, further comprises an additive, wherein the additive is selected from the group consisting of water proofing agents, emollients, preservatives, antioxidants, fragrances, humectants, rheology modifiers, aesthetic modifiers, vitamins, skin protectants, oils, emulsifiers, surfactants, pearlizers, consistency factors, thickeners, super fatting agents, stabilizers, polymers, silicone compounds, fats, waxes, lecithins, phospholipids, fillers, light management powders, antiperspirant actives (e.g., aluminum salts, zirconium salts) and mixtures thereof.

Preferably, the skin care formulation of the present invention has a pH of 4 to 9. More preferably, the skin care formulation of the present invention has a pH of 4.5 to 8.5. Still more preferably, the skin care formulation of the present invention has a pH of 5.0 to 8.0. Most preferably, the skin care formulation of the present invention has a pH of 5.5 to 7.5.

Some embodiments of the present invention will now be described in detail in the following **Examples.**

### Synthesis S1: Silylated Maltodextrin

Ammonium chloride (412.4 mg, 0.05 eq) and Glucidex^{®} 1 maltodextrin (DE 1 from Roquette) (25.0 g, 0.154 mol, 1.0 eq) were combined in a 2 CV Helicone mixer (CIT) with a stirring speed at 5 Hz. The resulting mixture was transferred to a reactor. Hexamethyldisilazane (80.87 g, 3.25 eq) was then added to the reactor contents dropwise. Dimethyl sulfoxide (10.8 g) was then added to the reactor contents. The reactor was then sealed and continuously flushed with nitrogen. The reactor contents were stirred at 20 Hz. Heat was applied to the reactor using a heating mantle set to a temperature of 40 °C and the stirring was increased to 50 Hz. After 30 min, the heating mantle was set to 50 °C. The temperature setting for the heating mantle was then increased to 80 °C over the course of 1 h by 10 °C increments. The reactor contents were stirred for 1 hr after the temperature of the reactor contents reached 71 °C. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~46.3 g). The degree of substitution, DS, of the -Si(CH₃)₃ on the maltodextrin base polymer was determined by¹H NMR to be 1.67.

### Synthesis S2: Silylated Maltodextrin

Ammonium chloride (454.4 mg, 0.05 eq) and Glucidex^{®} 1 maltodextrin (DE 1 from Roquette) (27.0 g, 0.166 mol, 1.0 eq) were combined in a 2 CV Helicone mixer (CIT) with a stirring speed at 5 Hz. The resulting mixture was transferred to a reactor. Hexamethyldisilazane (87.34 g, 3.25 eq) was then added to the reactor contents dropwise. Dimethyl sulfoxide (11.66 g) was then added to the reactor contents. The reactor was then sealed and continuously flushed with nitrogen. The reactor contents were stirred at 20 Hz. Heat was applied to the reactor using a heating mantle set to a temperature of 92 °C and the stirring was increased to 50 Hz. The reactor contents were stirred for 1.5 hr after the temperature of the reactor contents reached 83 °C. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~56.6 g). The degree of substitution, DS, of the -Si(CH₃)₃ on the maltodextrin base polymer was determined by ¹H NMR to be 2.1.

### Synthesis S3: Silylated Maltodextrin

Ammonium chloride (445.4 mg, 0.05 eq) and Glucidex^{®} 1 maltodextrin (DE 1 from Roquette) (27.0 g, 0.167 mol, 1.0 eq) were combined in a 2 CV Helicone mixer (CIT) with a stirring speed at 5 Hz. The resulting mixture was transferred to a reactor. Hexamethyldisilazane (60.47 g, 2.25 eq) was then added to the reactor contents dropwise. Dimethyl sulfoxide (11.7 g) was then added to the reactor contents. The reactor was then sealed and continuously flushed with nitrogen. The reactor contents were stirred at 20 Hz. Heat was applied to the reactor using a heating mantle set to a temperature of 55 °C and the stirring was increased to 50 Hz. After 5 min, the heating mantle was set to 102 °C. The reactor contents were stirred for 2 hr. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~407.3 g). The degree of substitution, DS, of the -Si(CH₃)₃ on the maltodextrin base polymer was determined by ¹H NMR to be 2.55.

### Synthesis S4: Silylated Maltodextrin

To a 25 mL scintillation vial was added ammonium chloride (33.0 mg, 0.05 eq) and Maltrin M250 maltodextrin (DE 23-27 from Grain Processing Corporation) (2.0 g, 12.3 mM, 1.0 eq). Hexamethyldisilazane (4.48 g, 2.25 eq) was then added dropwise to the vial contents. Dimethyl sulfoxide (1 g) was then added to the vial contents, and the vial was capped with a screw cap septum topped with two vent needles. The vial was placed on an aluminum heating block set at 85 °C for 1.5 hrs. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~4.15 g). The degree of substitution, DS, of the -Si(CH₃)₃ on the maltodextrin base polymer was determined by ¹H NMR to be 2.2.

### Synthesis S5: Silylated Cellulose

Polysaccharide (BioFloc XV, 15.0 g, from Tartas) was weighed in a 2 L three-neck flask equipped with a nitrogen inlet and a temperature controller. Solvent (N,N dimethylacetamide, 331 g, from Sigma-Aldrich) was added and the reaction mixture was placed under an atmosphere of nitrogen with an outlet to avoid over-pressurization of the reactor. Silane (hexamethyldisilazane, 30 g, from The Dow Chemical Company) was added at once to the reaction mixture. The mixture was slowly heated to a set temperature of 130 °C and stirred for 7.5 h. The solution was cooled naturally, then xylenes (600 g, from Sigma-Aldrich) was added to the reaction mixture along with additional hexamethyldisilazane (20 g) and the mixture was stirred for 4 h with a set temperature of 125 °C. The reactor contents were left to cool down to room temperature overnight. The reactor contents were then transferred to a separatory funnel and subjected to non-solvent precipitation by dropwise addition into 2 L of vigorously stirring methanol. The product was isolated by filtration, and dried in a vacuum oven at 50 °C overnight. The product was then suspended in 500 ml of methanol, then filtered and dried in a vacuum oven at 50 °C overnight. The product was analyzed by attenuated total reflection infrared to determine DS at 2.23.

### Synthesis S6: Silylated Cellulose

Polysaccharide (E-60, 15.2 g, from GP Cellulose) was weighed in a 2 L three-neck flask equipped with a nitrogen inlet and a temperature controller. Solvent (N,N dimethylacetamide, 324 g) was added and the reaction mixture was placed under an atmosphere of nitrogen with an outlet to avoid over-pressurization of the reactor. Silane (hexamethyldisilazane, 50.2 g, from The Dow Chemical Company) was added at once to the reaction mixture, along with the saccharin catalyst (850 mg, from Sigma-Aldrich). The mixture was slowly heated to a set temperature of 130 °C and stirred for 5 h. The solution was cooled naturally, then xylenes (400 g) were added to the reaction mixture and the mixture was stirred at 120 °C for 8 hours. The reactor contents were left to cool down to room temperature overnight. The cooled reactor contents were then transferred to a separatory funnel and subjected to non-solvent precipitation by dropwise addition into 2 L of vigorously stirring methanol. The product was isolated by filtration and dried in a vacuum oven at 50 °C overnight. The product was then suspended in 500 ml of methanol, then filtered again, and dried in a vacuum oven at 50 °C overnight. was analyzed by attenuated total reflection infrared to determine DS at 2.6.

### Synthesis S7: Silylated Maltodextrin

To a 25 mL scintillation vial was added ammonium chloride (33.0 mg, 0.05 eq) and Maltrin M200 maltodextrin (DE range 16.5-19.9 from Grain Processing Corporation)(2.0 g, 12.3 mM, 1.0 eq). Hexamethyldisilazane (3.58 g, 1.80 eq) was then added dropwise to the vial contents. Dimethyl sulfoxide (0.75 g) was then added to the vial contents and the vial was outfitted with a septum cap with two vent needles. The vial was placed on a heating block set at 80 °C for 1 hr. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~3.6 g). The degree of substitution, DS, of the -Si(CH₃)₃ on the maltodextrin base polymer was determined by ¹H NMR to be 2.76.

### Synthesis S8: Silylated Maltodextrin

To a 25 mL scintillation vial was added ammonium chloride (33.0 mg, 0.05 eq) and Maltrin M040 (DE 4-7 from Grain Processing Corporation) (2.0 g, 12.3 mM, 1.0 eq). Hexamethyldisilazane (4.48 g, 2.25 eq) was then added dropwise to the vial contents. Dimethyl sulfoxide (1 g) was then added to the vial contents and the vial was outfitted with a septum cap with two vent needles. The vial was placed on a heating block set at 85 °C for 1.5 hrs. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~4.15 g). The degree of substitution, DS, of the -Si(CH₃)₃ on the maltodextrin base polymer was determined by ¹H NMR to be 2.5.

### Synthesis S9: Silylated Maltodextrin

Ammonium chloride (445.4 mg, 0.05 eq) and Glucidex^{®} 1 maltodextrin (DE 1 from Roquette) (27.0 g, 0.166 mol, 1.0 eq) were combined in a 2 CV Helicone mixer (CIT) with a stirring speed at 5 Hz. The resulting mixture was transferred to a reactor. Hexamethyldisilazane (87.34 g, 3.25 eq) was then added to the reactor contents dropwise. Dimethyl sulfoxide (11.66 g) was then added to the reactor contents. The reactor was then sealed and continuously flushed with nitrogen. The reactor contents were stirred at 20 Hz. Heat was applied to the reactor using a heating mantle set to a temperature of 50 °C and the stirring was increased to 50 Hz. After 20 min, the heating mantle was set to 100 °C. The reactor contents were stirred for 2 hrs. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~53 g). The degree of substitution, DS, of the -Si(CH₃)₃ on the maltodextrin base polymer was determined by ¹H NMR to be 2.23.

### Synthesis S10: Silylated Maltodextrin

To a 25 mL scintillation vial was added ammonium chloride (33.0 mg, 0.05 eq) and Glucidex^{®} 1 maltodextrin (DE 1 from Roquette) (2.0 g, 12.3 mM, 1.0 eq). Hexamethyldisilazane (4.48 g, 2.25 eq) was then added dropwise to the vial contents. Dimethyl sulfoxide (1 g) was then added to the vial contents and the vial was outfitted with a septum cap with two vent needles. The vial was placed on a heating block set at 85 °C for 2 hrs. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~3.96 g). The degree of substitution, DS, of the -Si(CH₃)₃ on the maltodextrin base polymer was determined by ¹H NMR to be 2.51.

### Synthesis S11: Silylated Maltodextrin

Ammonium chloride (445.4 mg, 0.05 eq) and Glucidex^{®} 1 maltodextrin (DE 1 from Roquette) (27.0 g, 0.166 mol, 1.0 eq) were combined in a 2 CV Helicone mixer (CIT) with a stirring speed at 5 Hz. The resulting mixture was transferred to a reactor. Hexamethyldisilazane (87.34 g, 3.25 eq) was then added to the reactor contents dropwise. Dimethyl sulfoxide (11.66 g) was then added to the reactor contents. The reactor was then sealed and continuously flushed with nitrogen. The reactor contents were stirred at 20 Hz. Heat was applied to the reactor using a heating mantle set to a temperature of 50 °C and the stirring was increased to 50 Hz. After 20 min, the heating mantle was set to 100 °C. The reactor contents were stirred for 2 hrs. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~56.3 g). The degree of substitution, DS, of the -Si(CH₃)₃ on the maltodextrin base polymer was determined by ¹H NMR to be 2.42.

### Synthesis S12: Silylated Maltodextrin

To a 25 mL scintillation vial was added ammonium chloride (24.7 mg, 0.05 eq) and dried Glucidex^{®} 1 maltodextrin (DE 1 from Roquette) (1.5 g, 3.25 eq). Hexamethyldisilazane (4.85 g, 3.25 eq) was then added dropwise to the vial contents. Dimethyl sulfoxide (0.8 g) was then added to the vial contents and the vial was outfitted with a septum cap with two vent needles. The vial was placed on a heating block set at 90 °C for 4 hrs. The vial contents were then cooled down to < 50 °C and diluted with ethyl acetate (200 mL). The organic layer was transferred to a separatory funnel and washed with a 50/50 vol/vol mixture of brine and distilled water three times (3 x 60 mL). The organic layer was collected in an Erlenmeyer flask, and dried with sodium sulfate. The organic layer was then concentrated under vacuum to yield an off white crystalline solid, that was easily reduced to a fine powder with a spatula. The product powder was dried under vacuum in an oven at 50 °C for 5 hrs. The degree of substitution, DS, of the -Si(CH₃)₃ on the maltodextrin base polymer was determined by ¹H NMR to be 2.42.

### Synthesis S13: Silylated Maltodextrin

To a 25 mL scintillation vial was added ammonium chloride (24.7 mg, 0.05 eq) and dried Maltrin M150 maltodextrin (DE 13-17 from Grain Processing Corporation)(1.5 g, 1.0 eq). Hexamethyldisilazane (4.85 g, 3.25 eq) was then added dropwise to the vial contents. Dimethyl sulfoxide (0.8 g) was then added to the vial contents and the vial was outfitted with a septum cap with two vent needles. The vial was placed on a heating block set at 90 °C for 2.5 hrs. The vial contents were then cooled down to < 50 °C and diluted with ethyl acetate (200 mL). The organic layer was transferred to a separatory funnel and washed with a 50/50 vol/vol mixture of brine and distilled water three times (3 x 60 mL). The organic layer was collected in an Erlenmeyer flask, and dried with sodium sulfate. The organic layer was then concentrated under vacuum to yield an off white crystalline solid, that was easily reduced to a fine powder with a spatula. The product powder was dried under vacuum in an oven at 50 °C for 5 hrs. The degree of substitution, DS, of the -Si(CH₃)₃ on the maltodextrin base polymer was determined by ¹H NMR to be 2.64.

### Solubility screening (2 wt%)

The solubility of the products of **Syntheses S1-S6** and a commercial maltodextrin with a DE of 1 (Glucidex^{®} 1 from Roquette) were assessed in different carriers by combining individually in separate vials the products of **Syntheses S1-S6** (0.1 g) and the commercial maltodextrin with various solvents (4.9 g) as noted in **TABLE 1.** The resulting 2 wt% solutions were stirred with a magnetic stir bar for 1 hour at ~22 °C. The carriers and solubility observations are provided in **TABLE 1.**

**TABLE 1**

| **Carrier** | **Observation** | | | | | | |
|---|---|---|---|---|---|---|---|
| | **Solute (2 wt%) in carrier** | | | | | | |
| | **S1** | **S2** | **S3** | **S4** | **S5** | **S6** | **Malt⁹** |
| Water | I | I | I | I | I | I | S |
| Ethanol | I | S | S | S | I | I | I |
| Dimethicone, 2 cSt¹ | -- | S | S | S | I | S* | I |
| Isododecane | I | S | S | S | I | I | I |
| Isohexadecane | -- | S | S | -- | -- | -- | -- |
| Methyl undecanoate | -- | S | S | S | -- | -- | -- |
| Triglycerides² | -- | S | S | S | -- | -- | -- |
| Dicaprylyl carbonate³ | -- | S | S | S | -- | -- | -- |
| C₁₂₋₁₅ alkyl benzoate⁴ | -- | S | S | S | I | I | I |
| C₁₃₋₁₅ Alkane⁵ | -- | S | S | S | -- | -- | -- |
| Dioctvlether | -- | S | S | S | -- | -- | -- |
| Ethyl PG-Acetal Levulinate⁶ | -- | S | S | -- | -- | -- | -- |
| Homosalates⁷ | -- | S | S | S | -- | -- | -- |
| Sunflower oil⁸ | -- | I | I | S* | I | I | I |
| ¹ DOWSIL^{™} 200 fluid (2 cSt) from The Dow Chemical Company | | | | | | | |
| ² CRODAMOL^{™} GTCC from Croda | | | | | | | |
| ³ CETIOL^{®} CC from BASF | | | | | | | |
| ⁴ CRODAMOL^{™} ABLQ from Croda | | | | | | | |
| ⁵ NEOSSANCE^{®} hemisqualane from Centerchem | | | | | | | |
| ⁶ ECOSMOOTH^{™} universal fluid 1100 from The Dow Chemical Company | | | | | | | |
| ⁷ PARSOL^{®} HMS from DSM | | | | | | | |
| ⁸ Sunflower oil, USDA Certified Organic from MakingCosmetics | | | | | | | |
| ⁹ Glucidex^{®} 1 maltodextran with DE 1 from Roquette. | | | | | | | |
| ^{S} Soluble and clear | | | | | | | |
| ^{S*}Soluble and slightly hazy | | | | | | | |
| ^{I} Insoluble | | | | | | | |

### Solubility screening (50 wt%)

The solubility of the products of **Syntheses S2-S3** and **S7** (2 g) were assessed in isododecane (2 g) as noted in **TABLE 2.** The resulting 50 wt% solutions were stirred with a magnetic stir bar for 1 hour at ~22 °C. The carriers and solubility observations are provided in **TABLE 2.**

**TABLE 2**

| **Carrier** | **Observation** | | |
|---|---|---|---|
| | **Solute (50 wt%) in carrier** | | |
| | **S2** | **S3** | **S7** |
| Isododecane | S | S | S |
| Ethanol | not tested | S | S |
| ^{S} Soluble and clear | | | |

### Viscosity in isododecane

The product of **Synthesis S5-S6** and **S9** was dissolved in isododecane at different concentrations in as noted in **TABLE 3.** The viscosity of the resulting solutions were then determined using a Brookfield DV-111-ultra viscometer, equipped with a SC4-28 spindle at ~ 22 °C and 100 rpm. The results are provided in **TABLE 3.**

**TABLE 3**

| **Solute** | **Concentration (wt%)** | **Viscosity (cP)** |
|---|---|---|
| **Synthesis S5** | 2 | 3,500 |
| **Synthesis S6** | 2 | 3,200 |
| **Synthesis S6** | 5 | gel |
| **Synthesis S9** | 2 | 0 |
| **Synthesis S9** | 5 | 2 |
| **Synthesis S9** | 20 | 5 |
| **Synthesis S9** | 40 | 305 |
| **Synthesis S9** | 50 | 1,260 |

### Comparative Examples CF1-CF2 and Examples F1-F4: Pigmented dispersions

Pigmented dispersions were prepared in **Comparative Examples CF1-CF2** and **Examples F1-F4** by mixing Red Iron Oxide (CI 77491) (and) Triethoxycaprylylsilane pigment (1 g) from Gelest, a carrier (9.8 g) with ingredient (0.2 g) noted in **TABLE 4.**

**TABLE 4**

| **Example** | **Carrier** | **Ingrediant** |
|---|---|---|
| **Comp. Ex. CF1** | Isododecane | None |
| **Comp. Ex. CF2** | Isododecane | **Synthesis S7** |
| **Example F1** | Isododecane | **Synthesis S8** |
| **Example F2** | Isododecane | **Synthesis S9** |
| **Example F3** | Isododecane | **Synthesis S3** |
| **Example F4** | Isododecane | **Synthesis S10** |

### Rub off resistance

Films were deposited from the dispersions prepared according to **Comparative Examples CF1-CF2** and **Examples F1-F4** by coating on Vitro-skin (from IMS inc.) at 50 µm (wet thickness) using an automatic coater and a rectangular applicator. The deposited films were air dried in an environmental controlled room (~22 °C and 50% RH) overnight. The collagen was punched and adhered to an XRF cylindrical holder using double sided tape. Initial L, a, b values were measured using a color spectrophotometer from BYK-Gardner (Germany). The ΔE values for the films were calculated from the measured L, a and b values. The XRF cylinder was then placed on a felt band (thickener/film coating touching the band) and run through using a washability tester (Braive Instruments S.A., Belgium). The felt band was changed between each rub-off cycle. The results of the rub-off tests are provided in **TABLE 5.** The rub-off resistance (aka wear resistance) is directly related to the ΔE over rub off cycle. The lower the ΔE, the better the resistance to wear.

**TABLE 5**

| **Film of Example** | **Carrier** | **DE** | **DS** | **ΔE (at 50 cycles)** |
|---|---|---|---|---|
| **Comp. Ex. CF1** | Isododecane | -- | -- | 26.5 |
| **Comp. Ex. CF2** | Isododecane | 16.9-19.9 | 2.76 | 3.01 |
| **Example F1** | Isododecane | 4-7 | 2.5 | 2.10 |
| **Example F2** | Isododecane | 1 | 2.2 | 2.73 |
| **Example F3** | Isododecane | 1 | 2.55 | 2.10 |
| **Example F4** | Isododecane | 1 | 2.51 | 2.0 |

### Comparative Examples CF3-CF5 and Examples F5-F9: Water Repellency

Water repellency of a film is strongly influenced by surface energy. High water repellency is desirable for skin care applications. The water repellency of a formulation can be evaluated by measuring the water contact angle from a deposited film of the formulation. Specifically, films were coated onto a glass slide (50 µm) from dispersions formed using the ingredients noted in **TABLE 6** using a doctor blade film applicator with the gap set at 6 mils (0.1524 mm) from the as received polymer solutions, and films were air dried in an environmental controlled room (~22 °C and 50% RH) for at least 72 hours. The water contact angles for the deposited films were then measured (in degrees) at 4 and 120 seconds after water droplets were deposited on the substrate using a drop shape analyzer (Kruss DSA100). The results of the water contact angle measurements are provided in TABLE 6. Higher contact angles indicate greater water repellency. Contact angles of above 90° are considered excellent.

**TABLE 6**

| | **Ingredients** | | | **Water contact angle (°)** | |
|---|---|---|---|---|---|
| | **Polymer** | | **Isododecane** | | |
| **Example** | **Type** | **(wt%)** | **(wt%)** | **4 s** | **120 s** |
| **Comp. Ex. CF3** | Silicone Acrylate¹ | 2 | 98 | 104.5 | 102.3 |
| **Comp. Ex. CF4** | Modified pullulan² | 2 | 98 | 92.4 | 90.7 |
| **Comp. Ex. CF5** | Ethyl cellulose³ | 2 | 98 | 71.3 | 66.3 |
| **Example F4** | **Synthesis S11** | 2 | 98 | 96.2 | 94.2 |
| **Example F6** | **Synthesis S12** | 2 | 98 | 108.0 | 93.8 |
| **Example F7** | **Synthesis S13** | 2 | 98 | 106.1 | 101.5 |
| **Example F8** | **Synthesis S3** | 2 | 98 | 129.4 | 114.4 |
| **Example F9** | **Synthesis S3** | 20 | 80 | 112.6 | 100.2 |
| ¹ DOWSIL^{™} FA4012 ID acrylates/polytrimethylsiloxy-methacrylate copolymer from The Dow Chemical Company | | | | | |
| ² TSPL-30-ID isododecane (and) trimethylsiloxysilylcarbamoyl pullan from Shin-Etsu Chemical Co., Ltd. | | | | | |
| ³ ETHOCEL^{™} 7 from DuPont | | | | | |

### Comparative Examples CF6-CF9 and Examples F10-F12: Color cosmetic formulations

Color cosmetic formulations were prepared in **Comparative Examples CF6-CF9** and **Examples F10-F12** having the formulation noted in **TABLE 7.** The Phase A and Phase B ingredients were mixed in a separate container. The Phase B and Phase C ingredients were combined in a separate beaker and mixed until uniform. The mixed Phase B and C ingredients were then added to the combined Phase A ingredients and mixed until homogeneous.

**TABLE 7**

| **Phase** | **Ingredient INCI name** | **Example** | | | | | | |
|---|---|---|---|---|---|---|---|---|
| | | **CF6** | **CF7** | **CF8** | **CF9** | **F10** | **F11** | **F12** |
| | | **wt%** | | | | | | |
| A | PEG-10 Dimeticone¹ | 5 | 5 | 5 | 5 | 5 | 5 | 5 |
| A | Isododecane | 18.7 | 18.2 | 17.7 | 16.2 | 13.7 | 17.7 | 16.2 |
| A | Silica² | 2 | 2 | 2 | 2 | 2 | 2 | 2 |
| A | Silica silylate³ | 0.3 | 0.3 | 0.3 | 0.3 | 0.3 | 0.3 | 0.3 |
| A | Silicone elastomer blend⁴ | 6 | 6 | 6 | 6 | 6 | 6 | 6 |
| A | Product **Synthesis S9** | -- | -- | -- | -- | 5 | 1 | 2.5 |
| A | Product **Synthesis S6** | -- | 0.5 | 1 | 2.5 | -- | -- | -- |
| A | Isododecane (and) disteardimonium hectorite⁵ | 6 | 6 | 6 | 6 | 6 | 6 | 6 |
| B | Deionized water | 42 | 42 | 42 | 42 | 42 | 42 | 42 |
| B | Butylene glycol | 3 | 3 | 3 | 3 | 3 | 3 | 3 |
| B | Phenoxyethanol⁶ | 0.5 | 0.5 | 0.5 | 0.5 | 0.5 | 0.5 | 0.5 |
| B | Sodium chloride | 1 | 1 | 1 | 1 | 1 | 1 | 1 |
| C | Titanium dioxide⁷ | 15.5 | 15.5 | 15.5 | 15.5 | 15.5 | 15.5 | 15.5 |
| ¹ DOWSIL^{™} ES-5612 formulation aid from The Dow Chemical Company | | | | | | | | |
| ² Spheron P-1500 silica micro bead from Presperse | | | | | | | | |
| ³ DOWSIL^{™} VM-2270 aerogel fine particles from The Dow Chemical Company | | | | | | | | |
| ⁴ DOWSIL^{™} EL-TIPS silicone elastomer blend from The Dow Chemical Company | | | | | | | | |
| ⁵ Bentone ISD from Elementis Global | | | | | | | | |
| ⁶ Neolone PH 100 from DuPont | | | | | | | | |
| ⁷ ACT96-TRI-77891 from Miyoshi America | | | | | | | | |

### Rub off resistance

Films were deposited from the color cosmetic formulations prepared according to **Comparative Example CF6** and **Example F10** by coating on Vitro-skin (from IMS inc.) at 25 µm (wet thickness) using an automatic coater and a rectangular applicator. The deposited films were air dried in an environmental controlled room (~22 °C and 50% RH) overnight. The felt was punched and adhered to an XRF cylindrical holder using double sided tape. Initial L, a, b values were measured using a color spectrophotometer from BYK-Gardner (Germany). The ΔE values for the films were calculated from the measured L, a and b values. The XRF cylinder was then placed on a felt band (thickener/film coating touching the band) and run through using a washability tester (Braive Instruments S.A., Belgium). The felt band was changed between each rub-off cycle. The results of the rub-off tests are provided in **TABLE 8.** The rub off resistance (aka wear resistance) is directly related to the ΔE over rub off cycle. The lower the ΔE, the better the resistance to wear.

**TABLE 8**

| **Film of Example** | **ΔE after 50 cycles** |
|---|---|
| **Comp. Example CF6** | 17 |
| **Example F10** | 4 |

### Formulation Stability

The storage stability of the color cosmetic formulations prepared according to **Comparative Examples CF7-CF9** and **Examples F10-F12** were evaluated after six months of storage at room temperature (21 °C) and at 40 °C. The results are provided in **TABLE 9.**

**TABLE 9**

| **Formulation** | **Stable after six months** | |
|---|---|---|
| | **at 21°C** | **at 40** °**C** |
| **Comp. Example CF7** | ves | yes |
| **Comp. Example CF8** | yes | no |
| **Comp. Example CF9** | no | no |
| **Example F10** | yes | yes |
| **Example F11** | yes | yes |
| **Example F12** | yes | yes |

### Comparative Example CF10-CF12 and Example F13: Dispersions

Dispersions were prepared in **Comparative Examples CF10-CF12** and **Example** F13 by mixing isododecane (9.8 g) with ingredient (0.2 g) noted in **TABLE 10.**

**TABLE 10**

| **Example** | **Ingrediant** |
|---|---|
| **Comp. Ex. CF10** | Isododecane (and) Acrylates/Polytrimethylsiloxymethacrylate Copolymer¹ |
| **Comp. Ex. CF11** | Isododecane (and) Trimethylsiloxysilylcarbamoyl pullan² |
| **Comp. Ex. CF12** | Ethylcellulose³ |
| **Example F13** | product of **Synthesis S2** |
| ¹ DOWSIL^{™} FA4012 ID silicone acrylate from The Dow Chemical Company | |
| ² TSPL-30-ID from Shin-Etsu Chemical Co., Ltd. | |
| ³ ETHOCEL^{™} 7 from DuPont | |

### Water Repellency

Water repellency of a film is strongly influenced by surface energy. High water repellency is desirable for color cosmetic applications. The water repellency of a formulation can be evaluated by measuring the water contact angle from a deposited film of the formulation. Specifically, films were deposited from the dispersions prepared according to **Comparative Examples CF10-CF12** and **Example F13** on glass using a square film applicator with the gap set at 50 µm (wet thickness) from the as received polymer solutions, and films were air dried in an oven (~32 °C and 50% RH) for at least 16 hours. The water contact angles were measured (in degrees) at approximately 4 seconds and at 115 seconds after water droplets were deposited on the substrate using a drop shape analyzer (Kruss DSA100). The results of the water contact angle measurements are provided in **TABLE 11.** The contact angle retention reported in **TABLE 11** is according to the following formula: Retention (in %) = [(Contact Angle at time=115 sec.) / (Contact Angle at time=4 sec.)] * 100

**TABLE 11**

| **Composition** | **Water Contact angle** | | |
|---|---|---|---|
| | **time (in sec.)** | | **Retent. (%)** |
| | **4** | **200** | |
| **Comp. Example CF10** | 104.5 | 102.3 | 97.9 |
| **Comp. Example CF11** | 92.4 | 90.7 | 98.1 |
| **Comp. Example CF12** | 71.3 | 66.3 | 93.0 |
| **Example F13** | 96.2 | 94.2 | 97.9 |

## Claims

1. A skin care formulation, comprising:
a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 1 to 24; wherein the functionalized maltodextrin has a degree of substitution of -Si(R¹)₃ groups, DS, of 1.7 to 3; and wherein the functionalized maltodextrin is free of vinylic carbon.

2. The skin care formulation of claim 1, further comprising a dermatologically acceptable carrier.

3. The skin care formulation of claim 2, wherein the dermatologically acceptable carrier is selected from the group consisting of water; glycols; C₁₋₁₀ straight or branched chain alcohols; ketones; acetates; butyl cellusolve; dimethicones; polydimethylsiloxanes; alkanes; alkanoates, dermatologically acceptable hydrophobic ester oils; dicaprylyl carbonate; alkyl benzoates; hemisqualane; dioctylether; keto acids and mixtures thereof.

4. The skin care formulation of claim 3, further comprising a color ingredient.

5. The skin care formulation of claim 4, wherein the color ingredient is selected from the group consisting of inorganic pigments, organic pigments, aqueous pigment dispersions and mixtures thereof.

6. The color cosmetic formulation of claim 4, wherein the color ingredient is selected from the group consisting of Ext. D&C Yellow No. 2, Ext. D & C Violet No. 2, FD&C Red No. 4, FD&C Red No. 40, FD&C Yellow No. 5, FD&C Yellow No. 6, FD&C Green No. 3, FD&C Blue No. 1, D&C Yellow No. 7, D&C Yellow No. 8, D&C Yellow No. 10, D&C Yellow No. 11, D&C Violet No. 2, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 34, D&C Red No. 33, D&C Red No. 36, D&C Green No. 5, D&C Green No. 6, D&C Green No. 8, D&C Blue No. 4, D&C Orange No. 4, D&C Orange No. 5, D&C Orange No. 10, D&C Orange No. 11, D&C Brown No. 1, Aluminum powder, Annatto, Bismuth citrate, Bismuth Oxychloride, Bronze powder, Caramel, Carmine, β-Carotene, Chromium hydroxide green, Chromium oxide green, Copper chlorophyllin, Copper powder, Dihydroxyacetone, Ferric Ammonium ferrocyanide, Ferric ferrocyanide, Guanine, Iron oxide, Manganese Violet, Mica, Silver, Titanium Dioxide, Ultramarine, Zinc Oxide and mixtures thereof.

7. The color cosmetic formulation of claim 4, wherein the color ingredient includes at least one iron oxide.

8. The color cosmetic formulation of claim 7, wherein the dermatologically acceptable carrier includes isododecane.

9. The color cosmetic formulation of claim 4, further comprising a suncare active.

10. The color cosmetic formulation of claim 4, wherein the color cosmetic formulation has a pH of 5.5 to 7.5.

## Patentansprüche

1. Hautpflegeformulierung, umfassend:
ein filmbildendes Polymer, wobei das filmbildende Polymer ein funktionalisiertes Maltodextrin ist, umfassend ein mit -Si(R¹)₃-Gruppen funktionalisiertes Maltodextrin-Basispolymer; wobei jedes R¹ unabhängig eine lineare oder verzweigte gesättigte C₁₋₁₀-Alkylgruppe ist; wobei das Maltodextrin-Basispolymer ein Dextroseäquivalent, DE, von 1 bis 24 aufweist; wobei das funktionalisierte Maltodextrin einen Substitutionsgrad von -Si(R¹)₃-Gruppen, DS, von 1,7 bis 3 aufweist; und wobei das funktionalisierte Maltodextrin frei von vinylischem Kohlenstoff ist.

2. Hautpflegeformulierung nach Anspruch 1, ferner umfassend einen dermatologisch verträglichen Träger.

3. Hautpflegeformulierung nach Anspruch 2, wobei der dermatologisch verträgliche Träger aus der Gruppe ausgewählt ist, bestehend aus Wasser; Glykolen; geradkettigen oder verzweigten C₁₋₁₀-Alkoholen; Ketonen; Acetaten; Butylcellosolve; Dimethiconen; Polydimethylsiloxanen; Alkanen; Alkanoaten, dermatologisch verträglichen hydrophoben Esterölen; Dicaprylylcarbonat; Alkylbenzoaten; Hemisqualan; Dioctylether; Ketosäuren und Mischungen davon.

4. Hautpflegeformulierung nach Anspruch 3, ferner umfassend einen Farbbestandteil.

5. Hautpflegeformulierung nach Anspruch 4, wobei der Farbbestandteil aus der Gruppe ausgewählt ist, bestehend aus anorganischen Pigmenten, organischen Pigmenten, wässrigen Pigmentdispersionen und Mischungen davon.

6. Kosmetische Farbformulierung nach Anspruch 4, wobei der Farbbestandteil aus der Gruppe ausgewählt ist, bestehend aus Ext. D&C Gelb Nr. 2, Ext. D & C Violett Nr. 2, FD&C Rot Nr. 4, FD&C Rot Nr. 40, FD&C Gelb Nr. 5, FD&C Gelb Nr. 6, FD&C Grün Nr. 3, FD&C Blau Nr. 1, D&C Gelb Nr. 7, D&C Gelb Nr. 8, D&C Gelb Nr. 10, D&C Gelb Nr. 11, D&C Violett Nr. 2, D&C Rot Nr. 6, D&C Rot Nr. 7, D&C Rot Nr. 17, D&C Rot Nr. 21, D&C Rot Nr. 22, D&C Rot Nr. 27, D&C Rot Nr. 28, D&C Rot Nr. 30, D&C Rot Nr. 31, D&C Rot Nr. 34, D&C Rot Nr. 33, D&C Rot Nr. 36, D&C Grün Nr. 5, D&C Grün Nr. 6, D&C Grün Nr. 8, D&C Blau Nr. 4, D&C Orange Nr. 4, D&C Orange Nr. 5, D&C Orange Nr. 10, D&C Orange Nr. 11, D&C Braun Nr. 1, Aluminiumpulver, Annatto, Wismutcitrat, Wismutoxychlorid, Bronzepulver, Karamell, Carmin, β-Carotin, Chromhydroxidgrün, Chromoxidgrün, Kupferchlorophyllin, Kupferpulver, Dihydroxyaceton, Eisen(III)-ammoniumferrocyanid, Eisen(III)-ferrocyanid, Guanin, Eisenoxid, Manganviolett, Glimmer, Silber, Titandioxid, Ultramarin, Zinkoxid und Mischungen davon.

7. Kosmetische Farbformulierung nach Anspruch 4, wobei der Farbbestandteil mindestens ein Eisenoxid einschließt.

8. Kosmetische Farbformulierung nach Anspruch 7, wobei die dermatologisch verträgliche Trägersubstanz Isododecan einschließt.

9. Kosmetische Farbformulierung nach Anspruch 4, ferner umfassend einen Sonnenschutzwirkstoff.

10. Kosmetische Farbformulierung nach Anspruch 4, wobei die kosmetische Farbformulierung einen pH-Wert von 5,5 bis 7,5 aufweist.

## Revendications

1. Formulation pour soins de la peau, comprenant :
un polymère filmogène, dans laquelle le polymère filmogène est une maltodextrine fonctionnalisée comprenant un polymère de base de maltodextrine fonctionnalisé par des groupes -Si(R¹)₃ ; dans laquelle chaque R¹ est indépendamment un groupe alkyle saturé en C₁₋₁₀, linéaire ou ramifié ; dans laquelle le polymère de base de maltodextrine a un équivalent de dextrose, DE, de 1 à 24 ; dans laquelle la maltodextrine fonctionnalisée a un degré de substitution de groupes -Si(R¹)₃, DS, de 1,7 à 3 ; et dans laquelle la maltodextrine fonctionnalisée est exempte de carbone vinylique.

2. Formulation pour soins de la peau selon la revendication 1, comprenant en outre un support acceptable sur le plan dermatologique.

3. Formulation pour soins de la peau selon la revendication 2, dans laquelle le support acceptable sur le plan dermatologique est choisi dans le groupe constitué d'eau ; glycols ; alcools en C₁₋₁₀ à chaîne droite ou ramifiée ; cétones ; acétates ; butyl cellusolve ; diméthicones ; polydiméthylsiloxanes ; alcanes ; alcanoates, huiles d'esters hydrophobes acceptables sur le plan dermatologique ; carbonate de dicaprylyle ; benzoates d'alkyle ; hémisqualane ; dioctyléther ; cétoacides et mélanges de ceux-ci.

4. Formulation pour soins de la peau selon la revendication 3, comprenant en outre un ingrédient colorant.

5. Formulation pour soins de la peau selon la revendication 4, dans laquelle l'ingrédient colorant est choisi dans le groupe constitué de pigments inorganiques, pigments organiques, dispersions aqueuses de pigment et mélanges de ceux-ci.

6. Formulation de fard selon la revendication 4, dans laquelle l'ingrédient colorant est choisi dans le groupe constitué de Ext. D&C jaune n° 2, Ext. D&C violet n° 2, FD&C rouge n° 4, FD&C rouge n° 40, FD&C jaune n° 5, FD&C jaune n° 6, FD&C vert n° 3, FD&C bleu n° 1, D&C jaune n° 7, D&C jaune n° 8, D&C jaune n° 10, D&C jaune n° 11, D&C violet n° 2, D&C rouge n° 6, D&C rouge n° 7, D&C rouge n° 17, D&C rouge n° 21, D&C rouge n° 22, D&C rouge n° 27, D&C rouge n° 28, D&C rouge n° 30, D&C rouge n° 31, D&C rouge n° 34, D&C rouge n° 33, D&C rouge n° 36, D&C vert n° 5, D&C vert n° 6, D&C vert n° 8, D&C bleu n° 4, D&C orange n° 4, D&C orange n° 5, D&C orange n° 10, D&C orange n° 11, D&C marron n° 1, poudre d'aluminium, rocou, citrate de bismuth, oxychlorure de bismuth, poudre de bronze, caramel, carmin, β-carotène, vert d'hydroxyde de chrome, vert d'oxyde de chrome, chlorophylline de cuivre, poudre de cuivre, dihydroxyacétone, ferrocyanure d'ammonium ferrique, ferrocyanure ferrique, guanine, oxyde de fer, violet de manganèse, mica, argent, dioxyde de titane, bleu d'outremer, oxyde de zinc et mélanges de ceux-ci.

7. Formulation de fard selon la revendication 4, dans laquelle l'ingrédient colorant comporte au moins un oxyde de fer.

8. Formulation de fard selon la revendication 7, dans laquelle le support acceptable sur le plan dermatologique comporte de l'isododécane.

9. Formulation de fard selon la revendication 4, comprenant en outre un agent actif de soins solaires.

10. Formulation de fard selon la revendication 4, dans laquelle la formulation de fard a un pH de 5,5 à 7,5.
